# EUROPEAN PATENT APPLICATION

(11) **EP 3 928 727 A1**
(43) Date of publication of application: **29.12.2021**
(21) Application number: 21180608.8
(22) Date of filing: 21.06.2021
(51) Int. Cl.: A61B 17/74

(54) **MAIN NAIL FOR INTRAMEDULLARY NAIL SYSTEM AND THE INTRAMEDULLARY NAIL SYSTEM**

(30) Priority: 22.06.2020 CN 202010571709
(71) Applicant: Changzhou Kanghui Medical Innovation Co., Ltd., Changzhou, Jiangsu (CN)
(72) Inventor: KE, Linhua, Changzhou, 213100 (CN); QIN, Zongjun, Changzhou, 213100 (CN); LIU, Bo, Changzhou, 213100 (CN); PEI, Lei, Changzhou, 213100 (CN); GUAN, Jianjun, Changzhou, 213100 (CN); YANG, Zhaoquan, Changzhou, 213100 (CN)
(74) Representative: Müller-Boré & Partner Patentanwälte PartG mbB

(57) **Abstract**

The present disclosure generally relates to a main nail for an intramedullary nail system, and in particular, relates to a main nail for an intramedullary nail system for fixing a fractured lesser trochanter, which may also be used for fixing a fractured trochanter. A main nail for an intramedullary nail system is characterized by comprising a main nail, at the center of which a through-hole is disposed. At least one hole is provided at the proximal and distal sidewalls of the main nail for an intramedullary nail, the hole being used for accommodating a lag screw, a compression screw, or a securing and locking screw. The main nail for the intramedullary nail is provided with an oblique overlapping hole at the proximal end the overlapping hole being tapered from top to bottom. At the proximal outer wall, the main nail for the intramedullary nail is provided with an oblique arc groove along the lesser trochanter direction, a depth of the arc groove being less than its radius. The bottom plane where the arc groove is located does not interfere with an outer contour of an upper hole of the overlapping hole. Below the overlapping hole, the proximal end of the main nail for the intramedullary nail is provided with at least one proximal transverse locking screw hole. At least one locking screw hole is provided at the distal end of the main nail for the intramedullary nail.

## Description

### FIELD

The present disclosure generally relates to a main nail for an intramedullary nail system, and in particular relates to a main nail for an intramedullary nail system for fixing a fractured lesser trochanter. In addition, the present disclosure relates to an intramedullary nail system using the main nail. The main nail and the intramedullary nail system may also be used for fixing a fractured trochanter or femoral shaft.

### BACKGROUND

For a patient suffering from an intertrochanteric fracture, if there is also a fractured lesser trochanter, it is necessary to fix the fractured part of the lesser trochanter while reconstructing the femoral bone line. For the femoral nails currently available on the market, the locking screw hole in the lesser trochanter direction and the lag screw hole in the femoral direction both pass through the center plane of the main nail, and the two holes interfere in terms of space in the extending part of the lag screw along the direction of the femoral neck, making it impossible to simultaneously use the lag screw in the femoral neck direction and the screw in the lesser trochanter direction (i.e., only one of the them is allowed in use). When faced with a patient suffering from an intertrochanteric fracture along with a lesser trochanter fracture, a surgeon preferably selects the lag screw in the femoral direction and then uses a Kirschner wire for assisting in fixing the fractured lesser trochanter, thus reducing the dispersed bone fragments and restoring the support to the cortical bone at the inner wall of the femur. Constrained by the design of intramedullary nail prosthesis, this type of fracture in lesser trochanter area is not effectively secured, resulting in a potential risk of insufficient support to the inner sidewall and increasing the possibility of a secondary fracture after surgery. Furthermore, if the lesser trochanter is utterly fixed from the outside of the main nail during surgery, it is necessary to avoid the space occupied by the main nail in the medullary cavity when implanting a screw. In this way, there is significant damage to the unilateral femur of the patient and an iatrogenic fracture may occur easily. In particular, for a patient having a small proximal femur in diameter, the femur lacks sufficient bone support on one side to allow screw fixation.

### SUMMARY

In connection with the above defects of the intramedullary nail, an objective of the present disclosure is to provide a main nail for an intramedullary nail system. In particular, for a patient with an intertrochanteric fracture along with a lesser trochanter fracture, the femoral intramedullary nail according to the present disclosure can accomplish fixing of the fractured part of the lesser trochanter while reconstructing the femoral bone line. Moreover, the femoral intramedullary nail according to the present disclosure can be used to fix the intertrochanteric fracture or femoral shaft.

The main nail for an intramedullary nail system comprises a main nail body, an overlapping hole, and an arc groove. The main nail body includes a proximal portion, a distal portion, and an axial center through-hole. The overlapping hole is disposed at the proximal portion and obliquely passes through the main nail body in a radial direction. A side of the overlapping hole adjacent to a proximal end is greater than the other side adjacent to a distal end, and the two sides are configured to accommodate a lag screw and a compression screw, respectively. The arc groove is disposed on an outer wall of the proximal portion along a lesser trochanter direction and configured to accommodate a locking screw for fixing trochanter. An axis of the overlapping hole and an axis of the arc groove are non-planar, and the overlapping hole is configured to bypass the arc groove.

According to an aspect of the main nail for an intramedullary nail system as described herein, a depth of the arc groove is less than a radius of locking screw for fixing trochanter.

According to another aspect of the main nail for an intramedullary nail system as described herein, the main nail body comprises at least one proximal transverse locking screw hole disposed at a side of the overlapping hole adjacent to the distal end.

According to a further aspect of the main nail for an intramedullary nail system as described herein, a diameter of the proximal portion is greater than a diameter of the distal portion, and an included angle between an axis of the proximal portion and an axis of the distal portion is from 0 to 8 degrees.

According to a still further aspect of the main nail for an intramedullary nail system as described herein, an included angle between an axis of the overlapping hole and an axis of the proximal portion is from 120 to 140 degrees.

According to another aspect of the main nail for an intramedullary nail system as described herein, the arc groove is configured to bypass a thin-walled area around the overlapping hole and disposed at one side of the overlapping hole adjacent to the proximal end. In the cutting area at the proximal end of the main nail, the arc groove bypasses a thin-walled area of the overlapping hole formed at the proximal end of the main nail. The arc groove is preferably located closer to the tail end of the main nail for the intramedullary nail system. This area provides more clinical safety to guarantee that the fatigue strength of the main nail for the intramedullary nail system satisfies the clinical needs.

According to a further aspect of the main nail for an intramedullary nail system as described herein, the main nail comprises two proximal transverse locking screw holes, wherein one of the proximal transverse locking screw holes is an elongated hole while the other is a round hole. The elongated hole is closer to the overlapping hole than the round hole.

According to a still further aspect of the main nail for an intramedullary nail system as described herein, the main nail comprises three distal locking screw holes, wherein one of the distal locking screw holes is an elongated hole while the other two are round holes, the two rounded holes being identical in orientation. An included angle between an axis of the elongated hole and respective axis of the round holes is from 10 to 25 degrees.

According a further aspect of the main nail for an intramedullary nail system as described herein, the furthest round hole of the distal locking screw holes is a threaded hole, and the elongated hole is provided with a ridge.

To address the above defects of the intramedullary nail, a further objective of the present disclosure is to provide an intramedullary nail system. The intramedullary nail system comprises a main nail as mentioned above, a lag screw, and a locking screw for fixing trochanter. The lag screw can be disposed at a side of the overlapping hole adjacent to a proximal end, and the locking screw for fixing lesser trochanter can be disposed within the arc groove.

According to an aspect of the intramedullary nail system as described herein, the intramedullary nail system further comprises a compression screw which can be disposed at a side of the overlapping hole adjacent to a distal end.

According to an aspect of the intramedullary nail system as described herein, the intramedullary nail system further comprises at least one securing screw disposed within the proximal transverse locking screw holes.

According to an aspect of the intramedullary nail system as described herein, the intramedullary nail system further comprises at least one securing screw disposed within the distal transverse locking screw hole.

The present disclosure is used for a main nail for an intramedullary nail system and the intramedullary nail system, which has a reasonable design and a compact structure. The present disclosure is particularly suitable for a patient suffering from an intertrochanteric fracture along with a lesser trochanter fracture. The intramedullary nail according to the solution as described therein can accomplish the fixing of the fractured part of the lesser trochanter while reconstructing the femoral bone line.

In addition, the intramedullary nail according to the solution as described therein may also be employed for fixing a fractured trochanter or femoral shaft.

### BRIEF DESCRIPTION OF THE DRAWINGS

The present disclosure will be described below with reference to the drawings, wherein:
Fig. 1 is a schematic diagram illustrating a structure of a main nail for an intramedullary nail system according to the present disclosure;
Fig. 2 is a first schematic diagram illustrating a usage status of the main nail for the intramedullary nail system according to the present disclosure;
Fig. 3 is a second schematic diagram illustrating a usage status of the main nail for the intramedullary nail system according to the present disclosure;
Fig. 4 is a third schematic diagram illustrating a usage status of the main nail for the intramedullary nail system according to the present disclosure;
Fig. 5 is a fourth schematic diagram illustrating a usage status of the main nail for the intramedullary nail system according to the present disclosure.

The drawings contain the following reference symbols for respective features, including:
- 1: main nail,
- 2: lag screw,
- 3: compression screw,
- 6: fixing and locking screw,
- 7: locking screw for fixing trochanter,
- 11: main nail body,
- 12: overlapping hole,
- 13: center through-hole,
- 15: proximal locking screw hole,
- 16: arc groove,
- 17: distal locking screw hole,
- 121: upper hole,
- 122: lower low,
- 151: proximal elongated hole,
- 152: proximal round hole,
- 171: distal round hole,
- 172: distal elongated hole,
- 173: distal round threaded hole.

### DETAILED DESCRIPTION OF EMBODIMENTS

Reference will be made to the drawings hereinafter to specifically describe the technical solution according to the present disclosure, and the embodiments as described herein are only part of the embodiments of the present disclosure, rather than all of them. Other embodiments that those skilled in the art would acquire without creative work on the basis of the embodiments as described herein should all fall into the scope of the present disclosure.

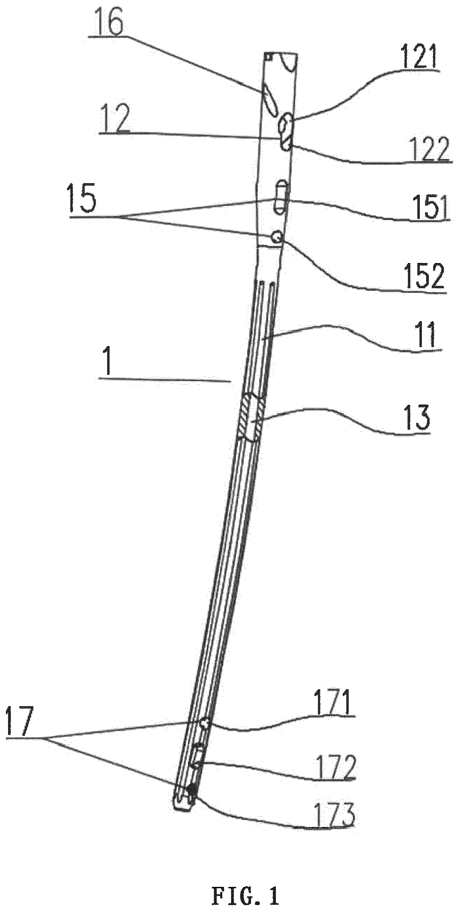

Referring to Figs. 1-4, the main nail for an intramedullary nail system includes a main nail 1. There is provided one through-hole 13 at the center of the main nail 1. The outer contour of the main nail 1 is thicker and straight at the proximal end while being thinner and arcuate at the distal end. An included angle of an axis of the proximal end and an axis of the distal end is from 0 to 8 degrees. The main nail 1 extends smoothly from the proximal end to the distal end. The intramedullary nail at the proximal and distal sidewalls is provided with at least one hole for receiving a lag screw 2, a compression screw 3, or a fixing and locking screw 6, respectively.

The main nail 1 is provided with an oblique overlapping hole 12 at the proximal end. The side of the overlapping hole 12 adjacent to the proximal end is greater than the other side adjacent to the distal end (i.e., so-called "tapered from top to bottom"). The overlapping hole 12 is comprised of an upper hole 121 and a lower hole 122, wherein an included angle between the axis of the overlapping hole 12 and the axis of the proximal end of the main nail for the intramedullary nail system is from 120 to 140 degrees, the upper hole 121 of the overlapping hole 12 may be mounted with the lag screw 2, and the lower hole 122 may be mounted with the compression screw 3.

The outer sidewall of the proximal end of the main nail 1 is provided thereon with an oblique arc groove 16 along the lesser trochanter direction. A bottom plane where the oblique arc groove 16 is located does not interfere with the outer contour of the upper hole 121 (i.e., the larger one) disposed at the upper part in the overlapping hole 12 in a space at the proximal end of the femur. In the cutting area at the proximal end of the main nail, the arc groove 16 bypasses a thin-walled area of the overlapping hole 12 formed at the proximal end of the main nail. The arc groove 16 is preferably located closer to the tail end of the main nail for the intramedullary nail system. This area provides more clinic safety to guarantee that the fatigue strength of the main nail 1 for the intramedullary nail system satisfies the clinical needs.

At the proximal end of the main nail 1, at least one proximal transverse locking screw hole (i.e., transverse hole) 15 is provided below the overlapping hole 12. The proximal transverse locking screw hole 15 is provided with an elongated hole 151 at the upper part and a round hole 152 at the lower part.

At the distal end of the main nail 1, there is provided at least one distal locking hole 17, preferably three distal locking screw holes 17, wherein two distal locking screw holes 171, 173 are identical in orientation, and a further distal locking screw hole 172 contains an angle of 10-20 degrees relative to respective axes of the other two distal locking screw holes. The furthest locking screw hole 173 is preferably configured as a round threaded hole and the distal locking screw hole 172 is preferably configured as an elongated hole and includes a ridge.

A threaded component is pre-installed within the center through-hole 13 at the proximal end of the main nail 1 and is provided for switching relative locating positions of the lag screw 2 and the main nail 1, i.e., a stably locked state and an unlocked state. The lag screw 2 is slidable in the main nail 1 along its axis in the unlocked state.

A tail cap for the proximal tail of the main nail 1 may be disposed within the center through-hole.

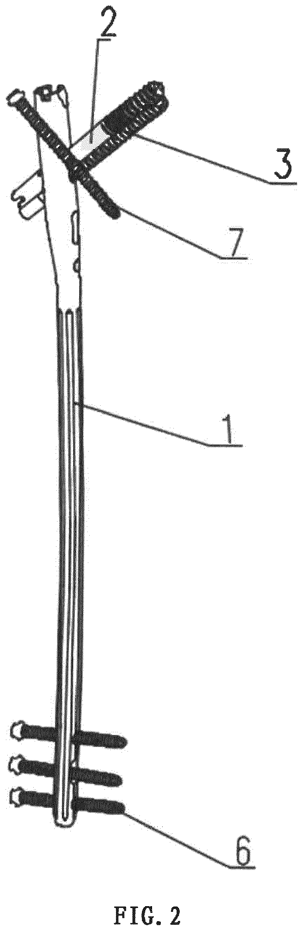

Embodiment 1: Fig. 2 is a first schematic diagram illustrating a usage status of the main nail for the intramedullary nail system. The illustrated main nail for the intramedullary nail system includes one main nail 1, one lag screw 2, one compression screw 3 and at least one fixing and locking screw 6. The lag screw 2 and the compression screw 3 are installed within an overlapping hole 12 at the proximal end of the main nail 1. The lag screw 2 and the compression screw 3 are installed sequentially and obliquely from bottom to top into respective appropriate depths. At this time, the compression screw 3 is driven to move the lag screw 2 smoothly rearwards along its axis, thereby achieving a compression effect at the fractured part.

In a further implementation, when a lesser trochanter is fractured, a locking screw 7 for fixing trochanter is installed into the arc groove 16 at the proximal end of the main nail 1 from outside to inside along the lesser trochanter direction, to fix dispersed fracture segments at the lesser trochanter site.

In a still further implementation, at least one fixing and locking screw 6 is installed within the locking screw hole 17 at the distal end of the main nail 1, to fix the main nail 1 within the patient's medullary cavity. Referring to Fig. 2, when three fixing and locking screws 6 are provided at the distal end, two of them are identical in orientation, and the middle one contains an angle of 10-25 degrees relative to the other two fixing and locking screws at the upper and lower sides in the same orientation in terms of axis. As such, a distal interlock is formed, thereby improving the stability of the main nail 1 within the medullary cavity.

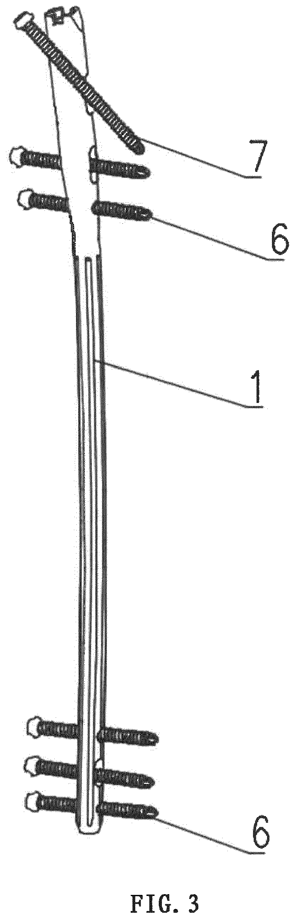

Embodiment 2: Fig. 3 is a second schematic diagram illustrating a usage status of the main nail for the intramedullary nail system. The main nail for the intramedullary nail system includes one main nail 1 and one fixing and locking screw 6.

In a further implementation, preferably at least one fixing and locking screw 6 is installed within the locking screw hole 17 at the distal end of the main nail 1, to fix the main nail at a distal end of a patient's medullary cavity. Referring to Fig. 3, when three fixing and locking screws 6 are provided at the distal end, two of them are identical in orientation, and the middle one contains an angle of 10-25 degrees relative to the other two fixing and locking screws at the upper and lower sides in the same orientation in terms of axis. As such, a distal interlock is formed, thereby improving the stability of the main nail 1 within the medullary cavity.

In a still further implementation, two fixing and locking screws 6 are installed within the proximal nail locking hole 15 of the intramedullary nail. The installment is implemented in the following order: first installing the fixing and locking screw 6 disposed above the proximal transverse locking screw hole 15, which is preferably located at a side adjacent to the upper edge of the elongated hole 151; and then installing the other fixing and locking screw 6 below the proximal locking screw hole 15 after the instrument completes the compression closure of the fracture part.

In a further more implementation, when a lesser trochanter is fractured, a locking screw 7 for fixing trochanter is installed within the arc groove 16 at the proximal end of the main nail 1 for the intramedullary nail system from outside to inside along the lesser trochanter direction, to fix dispersed fracture segments at the lesser trochanter site.

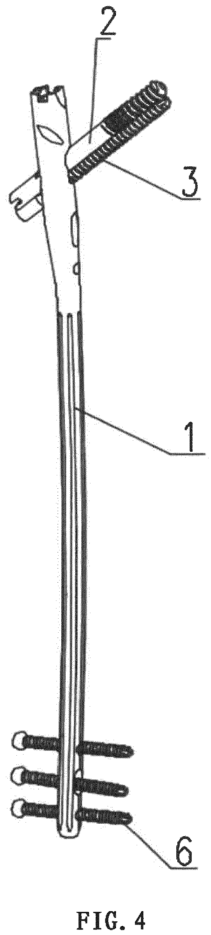

Embodiment 3: Fig. 4 is a third schematic diagram illustrating a usage status of the main nail for the intramedullary nail system according to the present disclosure. The main nail for the intramedullary nail system includes one main nail 1, one lag screw 2, one compression screw 3, and one fixing and locking screw 6. The lag screw 2 and the compression screw 3 are installed within an overlapping hole 12 at the proximal end of the main nail 1, specifically installed sequentially and obliquely from bottom to top into respective anticipated depths.

In a further implementation, at the distal end of the main nail 1, preferably at least one fixing and locking screw 6 is installed within the locking screw hole 17, to fix the main nail 1 for the intramedullary nail system at a distal end in a patient's medullary cavity. Referring to Fig. 4, when three fixing and locking screws 6 are provided at the distal end, two of them are identical in orientation, and the middle one contains an angle of 10-25 degrees relative to the other two fixing and locking screws at the upper and lower sides in the same orientation in terms of axis. As such, a distal interlock is formed, thereby improving the stability of the main nail 1 within the medullary cavity.

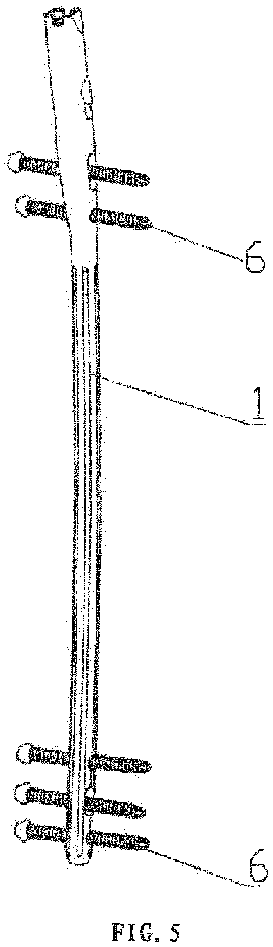

Embodiment 4: Fig. 5 is a fourth schematic diagram illustrating a usage status of the main nail for the intramedullary nail system. The main nail for the intramedullary nail system includes one main nail 1 and one fixing and locking screw 6, which is suitable for fixing a fractured femoral shaft.

In a further implementation, at the distal end of the main nail 1, preferably at least one fixing and locking screw 6 is installed within the locking screw hole 17, to fix the main nail 1 at a distal end in a patient's medullary cavity. Referring to Fig. 5, when three distal fixing and locking screws 6 are provided, two of them are identical in orientation, and the middle one contains an angle of 10-25 degrees relative to the other two fixing and locking screws at the upper and lower sides in the same orientation in terms of axis. As such, a distal interlock is formed, thereby improving the stability of the main nail 1 within the medullary cavity.

In a still further implementation, two fixing and locking screws 6 are installed within the locking screw hole 15 at the proximal end of the intramedullary nail. The installment is implemented in the following order: first installing the fixing and locking screw 6 above the transverse locking screw hole 15, which is preferably located at a side adjacent to the upper edge of the elongated hole 151, and then installing the other fixing and locking screw 6 below the locking screw hole 15 at the proximal end after the instrument completes the compression closure of the fracture end.

## Claims

1. A main nail for an intramedullary nail system, comprising:
a main nail body (11) comprising a proximal portion, a distal portion, and an axial center through-hole (13);
an overlapping hole (12) disposed at the proximal portion and obliquely passing through the main nail body (11) in a radial direction, a side (121) of the overlapping hole (12) adjacent to a proximal end being greater than the other side (122) adjacent to a distal end, the two sides being configured to accommodate a lag screw (2) and a compression screw (3), respectively; and
an arc groove (16) disposed on an outer wall of the proximal portion along a lesser trochanter direction and configured to accommodate a locking screw (7) for fixing trochanter;
wherein an axis of the overlapping hole (12) and an axis of the arc groove (16) are non-coplanar, and the overlapping hole (12) is configured to bypass the arc groove (16).

2. The main nail for an intramedullary nail system of claim 1, wherein a depth of the arc groove (16) is less than a radius of the locking screw (7) for fixing trochanter.

3. The main nail for an intramedullary nail system of claim 2, wherein the main nail body (11) comprises at least one proximal transverse locking screw hole (151, 152) disposed at a side of the overlapping hole (12) adjacent to the distal end.

4. The main nail for an intramedullary nail system of any of claims 1-3, wherein a diameter of the proximal portion is greater than a diameter of the distal portion, and an included angle between an axis of the proximal portion and an axis of the distal portion is from 0 to 8 degrees.

5. The main nail for an intramedullary nail system of claim 4, wherein an included angle between an axis of the overlapping hole (12) and an axis of the proximal portion is from 120 to 140 degrees.

6. The main nail for an intramedullary nail system of claim 5, wherein the arc groove (16) is configured to bypass a thin-walled area around the overlapping hole (12) and disposed at one side of the overlapping hole (12) adjacent to the proximal end.

7. The main nail for an intramedullary nail system of claim 6, wherein two of the proximal transverse locking screw holes (151, 152) are provided, wherein one of the proximal transverse locking screw holes (151, 152) is an elongated hole while the other is a round hole, the oval hole being closer to the overlapping hole (12) than the round hole.

8. The main nail for an intramedullary nail system of claim 7, where three distal locking screw holes (171, 172, 173) are provided, wherein one of the distal transverse locking screw holes (171, 172, 173) is an elongated hole while the other two are round holes, the two round holes being identical in orientation, and an included angle between an axis of the elongated hole and respective axis of the round holes is from 10 to 25 degrees.

9. The main nail for an intramedullary nail system of claim 8, wherein the furthest round hole of the distal locking screw holes (171, 172, 173) is a threaded hole, and the elongated hole is provided with a ridge.

10. An intramedullary nail system, comprising a main nail (1) of any of the preceding claims, a lag screw (2), and a locking screw (7) for fixing trochanter, wherein the lag screw (2) can be disposed at a side (121) of the overlapping hole (12) adjacent to a proximal end, and the locking screw (7) for fixing trochanter can be disposed within the arc groove (16).

11. The intramedullary nail system of claim 10, further comprising a compression screw (3) which can be disposed at a side (122) of the overlapping hole (12) adjacent to a distal end.

12. The intramedullary nail system of claim 11, further comprising at least one fixing screw (6) disposed within the proximal transverse locking screw holes (151, 152).

13. The intramedullary nail system of claim 12, further comprising at least one fixing screw (6) disposed within the distal transverse locking screw hole (171, 172, 173).
